# EUROPEAN PATENT APPLICATION

(11) **EP 4 696 267 A2**
(43) Date of publication of application: **18.02.2026**
(21) Application number: 25220386.4
(22) Date of filing: 21.06.2023
(51) Int. Cl.: A61B 18/18

(54) **HAIR TREATMENT DEVICE**

(30) Priority: 28.06.2022 EP 22181526
(62) Divisional of application: 23733975.9
(71) Applicant: Koninklijke Philips N.V., 5656 AG Eindhoven (NL)
(72) Inventor: BOAMFA, Marius Iosif, 5656 AG Eindhoven (NL); CHEN, Chen, 5656 AG Eindhoven (NL); VAN GOMPEL, Anton, 5656 AG Eindhoven (NL)
(74) Representative: Philips Intellectual Property & Standards

(57) **Abstract**

A hair treatment device comprises an IPL lamp driven with pulses of energy. A switch is in series with the IPL lamp and is switched at a frequency in the range 50 kHz to 500 kHz to provide pulsed delivery of current to the IPL lamp with pulses shorter than the overall IPL pulse duration The current modulation is used to eliminate the initial current peak and its associated peak in light output. The modulation can be used during the entire IPL pulse, or only during a fraction of it, while the duty cycle or the frequency of the modulation can change during the IPL pulse in order to optimize the light output. A pulsed delivery of current is provided to the IPL lamp during a first portion of the pulses of energy and a continuous delivery of current is provided during a last portion of the pulses of energy.

## Description

### FIELD OF THE INVENTION

This invention relates to hair treatment devices, in particular for hair removal or hair growth reduction by photo-epilation.

### BACKGROUND OF THE INVENTION

Photo-epilation is well known for hair removal and hair growth reduction. Home-use consumer devices are commercially available, such as the Lumea (TM) of Philips (TM). Home-use devices typically use Intense Pulsed Light technology (IPL) from e.g. a Xenon flash lamp at a relatively low fluence (up to 6.5 J/cm²), as compared to professional devices for permanent photo-epilation, that use fluences in excess of 10 J/cm².

IPL technology uses a high-powered, hand-held, flash lamp to deliver an intense, visible, broad-spectrum pulse of light, generally in the visible spectral range of 400 to 1200 nm. Cutoff filters are for example used to selectively filter out shorter wavelengths, especially potentially damaging ultra violet light. The resulting light has a spectral range that targets specific structures and chromophores, in particular the melanin pigment in hair. IPL shares some similarities with laser treatments, in that they both use light to heat and destroy their targets. Unlike lasers that use a single wavelength of light which typically matches only one chromophore and hence only treats one condition, IPL uses a broad spectrum.

The light absorbed by melanin in the hair and hair matrix generates heat that damages the hair follicles. When the treatment is repeated in intervals of 2 to 4 weeks, a long-lasting hair reduction result is obtained.

US2012/0010684A1 discloses a dermatologic treatment device comprising a flashlamp, a pulse-drive circuit which provides electrical energy to pulse the flashlamp, and a control circuit which selectively enables transmission of electrical energy to the flash lamp based on a signal having duty cyle indicative of when an AC line voltage exceeds a minimum operating voltage threshold. The circuit comprises a switch in series with the flash lamp controlled e.g. at a frequency 50 kHz to 100 kHz.

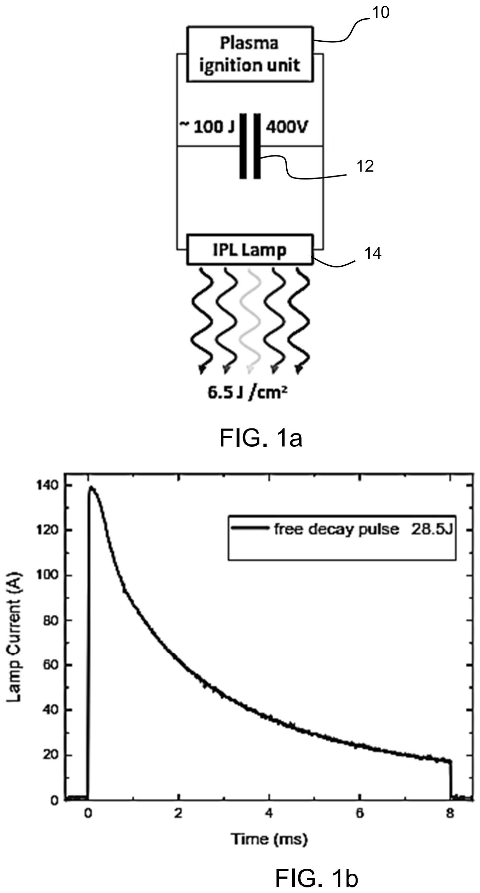

Fig. 1a shows the known pulse generation circuitry and Fig. 1b shows the IPL pulse (as current versus time), as implemented in the Lumea (TM) system.

Plasma is ignited by a plasma ignition unit 10. Energy is stored in a main storage capacitor 12 (e.g. 100J of energy at 400V). The electrical energy stored in the capacitor is discharged through the lamp 14, resulting in a pulsed operation of the lamp. The lamp is for example a gas-filled linear flash lamp.

The plasma ignition unit 10 generates a conductive plasma channel of ionized gas atoms or molecules between the lamp electrodes (inside the lamp tube). At first, a high voltage (of around 15kV) is applied between the lamp electrodes. The high voltage is needed to generate the electrical breakdown of the gas inside the lamp. The high voltage cannot however deliver the electrical charge needed to open a conductive plasma channel.

For this, a boost capacitor may be used. This boost capacitor delivers the required electrical charge. It only stores a small amount of electrical energy, about 1-2% of the electrical energy compared to the main capacitor, while its electrical voltage is around 800V.

The timing sequence of the high voltage, boost capacitor discharge and the main capacitor discharge are very precise for the lamp discharge to take place. In particular, the three steps overlap; the boost capacitor discharge starts while the gas breakdown is still present and the main capacitor discharge is initiated before the boost capacitor discharge is finished.

The lamp is for example fitted within a head of the hair treatment device with a radiation exit opening. During operation, the lamp generates light pulses having a relatively high energy density, which propagate towards the radiation exit opening and irradiate the human skin present in front of the radiation exit opening. Part of the light is absorbed by the hair roots and the hair follicles present in the skin, which are considerably heated as a result of the relatively high energy density of the light. As a result the hair roots and the hair follicles are damaged or even destroyed, so that growing of the hairs is prevented for a considerably long time or even permanently.

As shown by the IPL pulse shape, the current rises very rapidly to its maximum and then decays exponentially as the energy in the capacitor is consumed and the light pulse is generated. This type of discharge is an exponential decay pulse, as the current (as well as the light intensity output of the IPL lamp) follows the form of an exponential decay during the IPL flash. In the example shown, the current is stopped after 8ms, by means of a power transistor.

This method has the advantage of an easy implementation, as it is cost effective, and a minimal physical volume is occupied by the used components. However, the large light output intensity at the beginning of the pulse (caused by the initial lamp current peak) can create discomfort for the user. Some users describe the IPL flash as painful and some others as a sharp unpleasant sting.

It has been recognized that it would be desirable make the light output during the IPL pulse more even, ideally with a so-called block pulse, in particular by adapting the lamp current so that it is more constant over the duration of the IPL pulse, instead of having a high initial peak shown in Fig. 1b.

Fig. 2a shows the addition of control electronics 20 between the energy storage capacitor 12 and the lamp 14, and shows the desired block pulse 22 in Fig. 2b. The more uniform lamp current during the IPL pulse is beneficial for the user comfort. It also opens the way for treatment personalization and treatment efficiency benefits, as proven in professional IPL devices.

However, known or contemplated implementations of the block pulse discharge to generate the IPL flash are not cost effective and the components take more space in the device as compared to the case of the free discharge implementation.

Fig. 3 shows a first possible implementation concept of the block pulse discharge for the IPL flash, using a coil 30 and current modulation.

The storage capacitor 12 is coupled to the lamp and coil in series by a first switch 16, and a second switch 18 is in parallel with the combination of the lamp and coil. The operation of the two switches 16, 18 is coordinated, such that one is open when the other one is closed (and vice versa). In this way, the coil is used as a current smoothing circuit, and it is alternately loaded with energy and then releases energy to the IPL lamp.

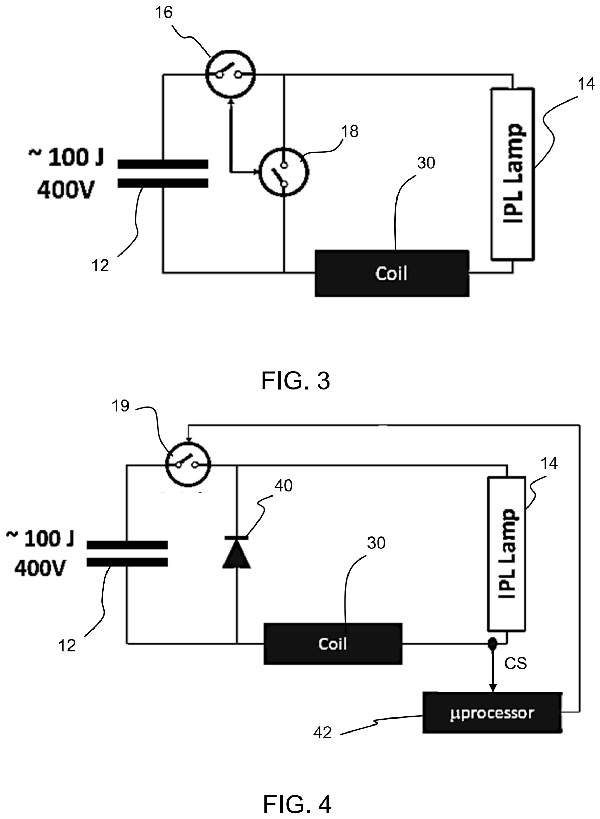

Fig. 4 shows a second possible implementation concept of the block pulse discharge for the IPL flash, using a coil 30, a power diode 40 and a controller 42 (microprocessor) that controls the current modulation scheme using a switch 19 in series with the IPL lamp, using a current feedback signal CS provided by a current sensor.

These approaches both make use of a large physical coil 30 forming part of a current regulating circuit, with the main drawbacks of a large physical space needed in the device as well as a high cost solution. A magnetic material core coil cannot be used as it may face the issue of saturation due to the large current and/or the high switching frequency. Therefore an air core coil is needed, because an air core coil does not saturate. However it takes a large space due to its low inductance.

There is therefore a need for a cost-effective and space-efficient solution to the above-mentioned user discomfort caused by the IPL exponential decay pulse.

### SUMMARY OF THE INVENTION

The invention is defined by the claims.

According to examples in accordance with an aspect of the invention, there is provided a hair treatment device comprising:
an intense pulsed light, IPL, lamp;
a drive circuit for driving the IPL lamp with pulses of energy with first duration, wherein the drive circuit comprises an ignition unit and a main storage capacitor;
a switch in series with the IPL lamp; and
a controller for controlling the switch at a frequency in the range 50 kHz to 300 kHz thereby to provide pulsed delivery of current to the IPL lamp, during at least a portion of the pulses of energy, with current delivery pulses of a second duration shorter than the first duration.

This device provides a high frequency modulation of the IPL lamp current, in particular in the 50 kHz to 500 kHz range. This modulation is used to eliminate the initial current peak and its associated peak in light output.

The high frequency modulation of the lamp current can be used for the full duration of the pulses of energy, or only for a fraction of the pulses of energy. For example, a first portion (e.g. half) of the pulses of energy have current modulation whereas the last portion (e.g. half) of the pulses of energy have no current modulation, i.e. a continuous delivery of current and hence a free decay, after the initial current peak is eliminated.

The current peak reduction can be achieved with no extra inductive or capacitive elements. Thus, small capacitor and inductor components may be used if such components are desired for circuit optimization. The modulation provided by the switch can be used during the entire IPL pulse (i.e. the first duration), or only during a fraction of it.

The lower value of 50 kHz represents the switching frequency below which the light intensity is still found to follow an exponential (but pulsed) decay, and still with a high initial light intensity. The upper value of 500 kHz limits the impact of the lifetime of the circuit components (which is worse at higher frequencies), as well as limiting the switching losses and thermal load.

A preferred frequency is in the range 100kHz to 300kHz, for example 100kHz to 250kHz, or 150kHz to 300kHz, or 150kHz to 250kHz.

The first duration is for example in the range 5 ms to 10 ms, such as 8ms. The IPL pulses are hence much longer than the switch control pulses, namely the second duration defined above, (e.g. 100 kHz corresponds to 10 µs pulse period and hence a 5µs pulse duration for a 50% duty cycle).

The duty cycle and the frequency of the pulsed delivery of current may be fixed, or they be adjusted to compensate for lamp aging effects, for example using analysis of past flashes.

The controller may be configured to adjust the frequency over time during the pulses of energy. The controller is for example alternatively or additionally configured to adjust a duty cycle of the control of the switch over time during the pulses of energy.

These measures enable the light output characteristics over the duration of the IPL pulses to be optimized.

The controller is for example configured to increase the duty cycle from a first value in the range 20% to 40% to a second value in the range 40% to 70% over time during the pulses of energy. This is found to enable a more flat light intensity profile to be generated.

The device may further comprise a diode electrically in parallel with the IPL lamp with the anode of the diode connected to a low voltage terminal of the IPL lamp and the cathode of the diode connected to a high voltage terminal of the IPL lamp. This diode provides a conduction path (in an opposite direction to the lamp current) which enables the device to make use of any residual inductance that the lamp might feature during the modulation.

The device may further comprise a secondary capacitor in parallel with the IPL lamp. This secondary capacitor can charge while the main capacitor output is on, while discharging over the lamp when the main capacitor is off. Thus, it provides a further smoothing function.

The secondary capacitor for example has a capacitance below 1% of the capacitance of the main capacitor. Thus, it does not occupy a significant space or introduce a significant cost.

In another example, the device further comprises a diode and a secondary capacitor in series with each other and together electrically in parallel with the IPL lamp, and an inductor between a high voltage terminal of the IPL lamp and a junction between the diode and the secondary capacitor.

The secondary capacitor preferably has a capacitance below 1% of the capacitance of the main capacitor and the inductor has an inductance in the range 1 mH to 100 mH. Thus, they do not occupy a significant space or introduce a significant cost.

The IPL lamp for example comprises a flash lamp with a fluence below 6.5 J/cm². Thus, the device is suitable as a home-use consumer device.

The device may further comprise a current sensor or a light intensity sensor for providing a feedback signal to the controller. In this way, feedback may be used to regulate the drive current or light output.

The controller is for example configurable to operate the device in first and second modes, a first mode with said current delivery pulses during said pulses of energy, and a second mode with delivery of continuous current during said pulses of energy. The first mode is for example particularly suitable for sensitive skin areas (e.g. the armpits) whereas the second mode may be used for less sensitive skin areas (e.g. the legs). The switching between modes may be manually controlled by a user of the device, or it may be automatic based on sensing inputs.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows known pulse generation circuitry and an IPL pulse (as current versus time);

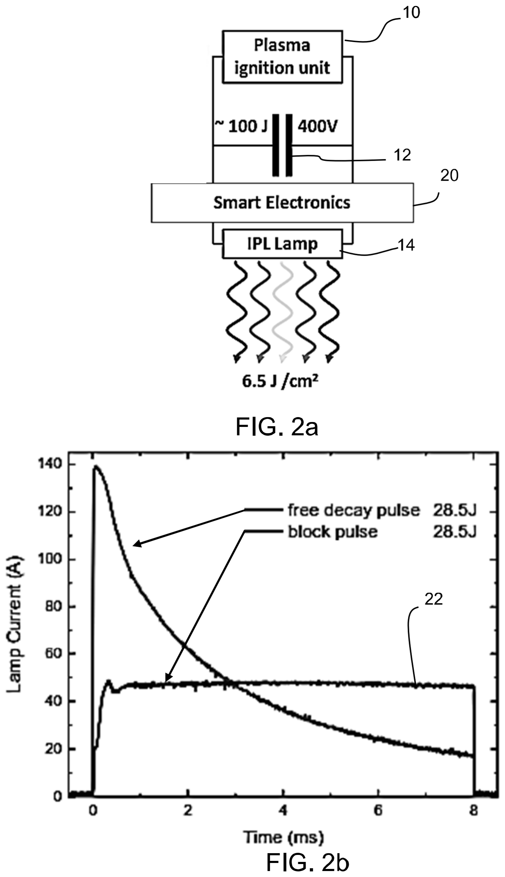

Fig. 2 shows the addition of control electronics between the energy storage capacitor and the lamp and shows a desired block pulse;
Fig. 3 shows a first possible implementation concept of the block pulse discharge for the IPL flash, using a coil and current modulation;
Fig. 4 shows a second possible implementation concept of the block pulse discharge for the IPL flash, using a coil, a power diode and a controller;

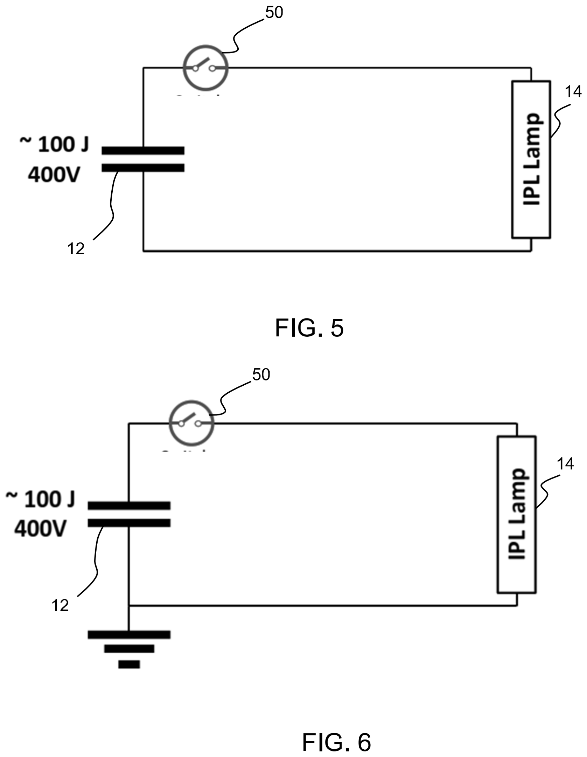

Fig. 5 shows a first example of a circuit using the approach of the invention;
Fig. 6 shows a first implementation of the circuit of Fig. 5;

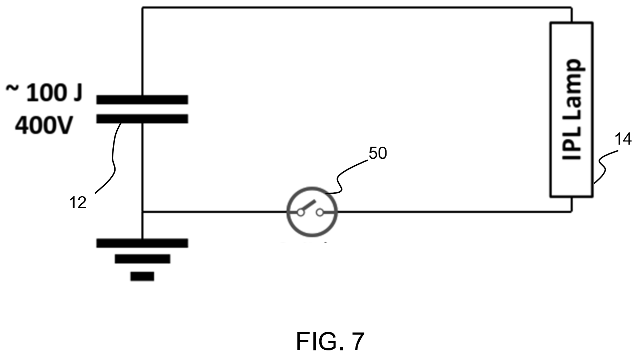

Fig. 7 shows a second implementation of the circuit of Fig. 5;

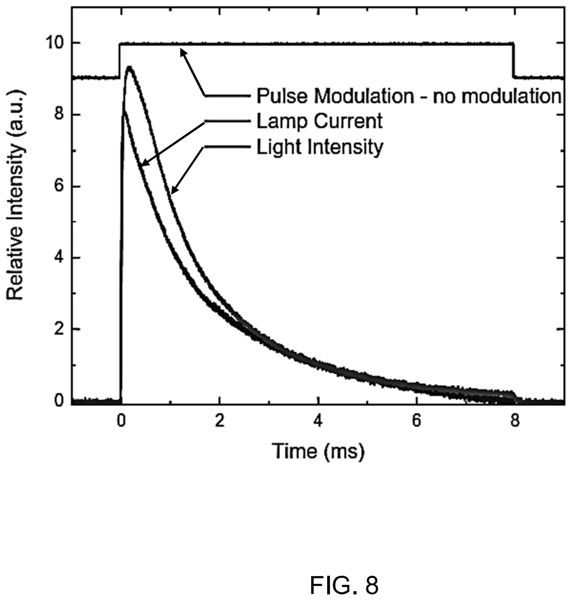

Fig. 8 shows the lamp current and light intensity (i.e. light output) during an unmodulated free decay discharge of an IPL device.

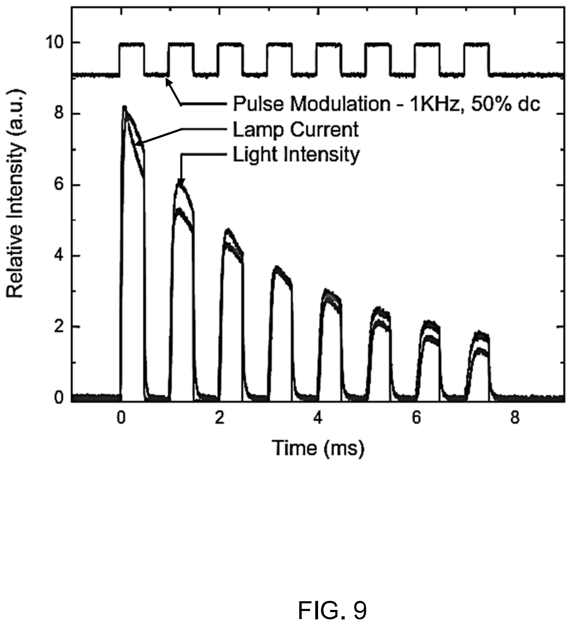

Fig. 9 shows the lamp current and light intensity when a 1 kHz, 50% duty cycle modulation is introduced, using the configuration presented in Fig. 5;

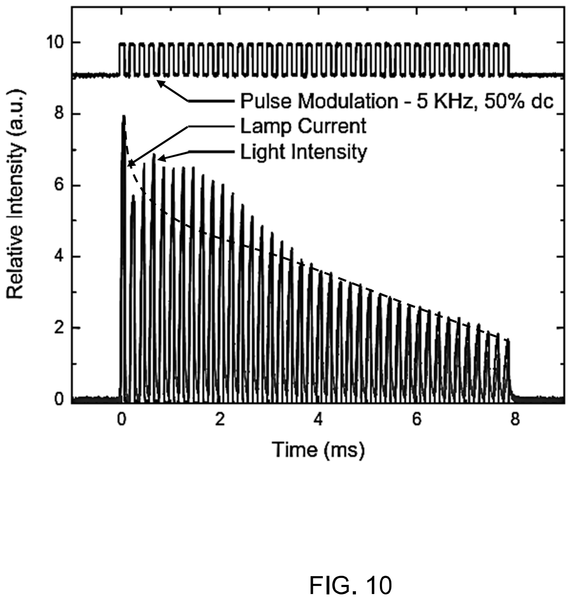

Fig. 10 shows the current and light output during a 5 kHz, 50% duty cycle over a long time scale;

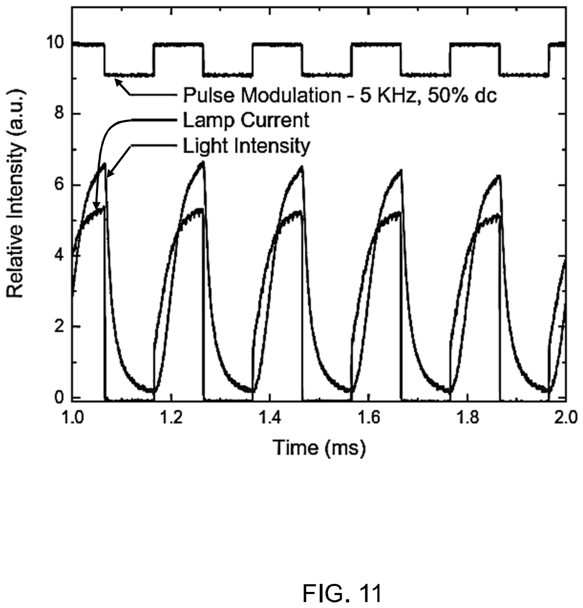

Fig. 11 shows the current and light output during a 5 kHz, 50% duty cycle, over a short time scale;

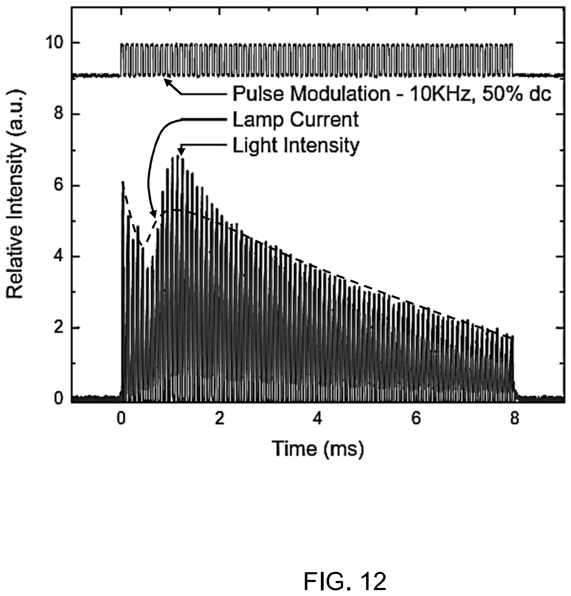

Fig. 12 shows the current and light output during a 10 kHz, 50% duty cycle over a long time scale;

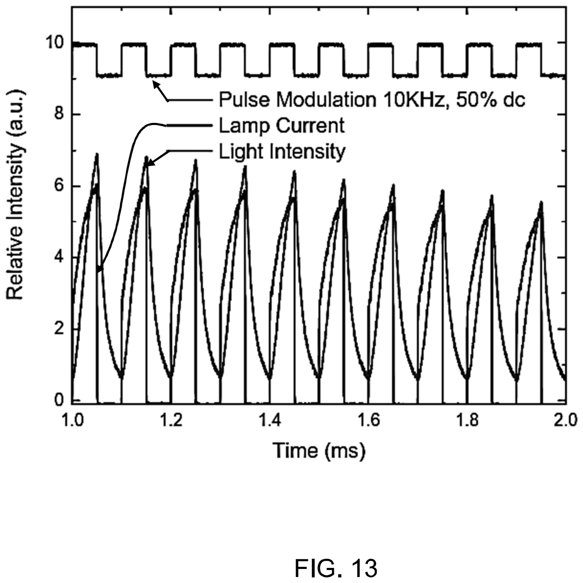

Fig. 13 shows the current and light output during a 10 kHz, 50% duty cycle over a short time scale;

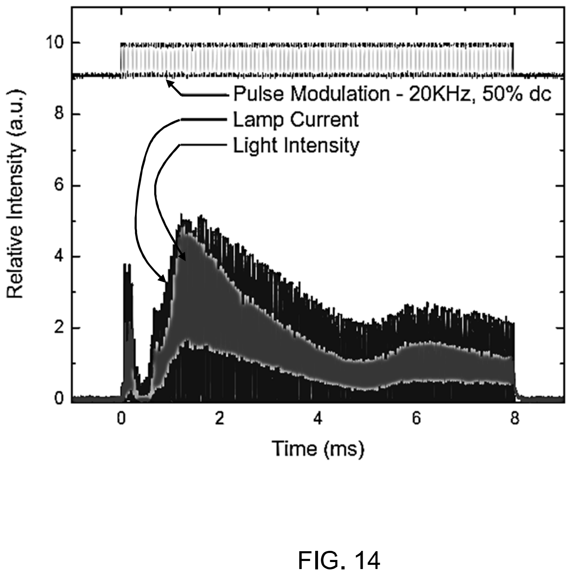

Fig. 14 shows the current and light output during a 20 kHz, 50% duty cycle over a long time scale;

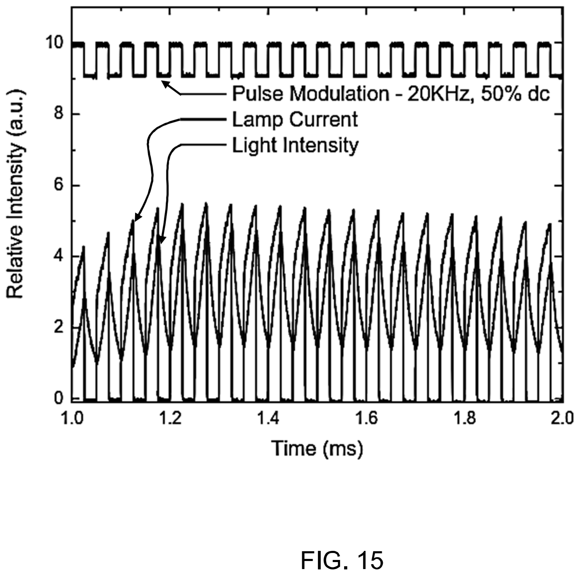

Fig. 15 shows the current and light output during a 20 kHz, 50% duty cycle over a short time scale;

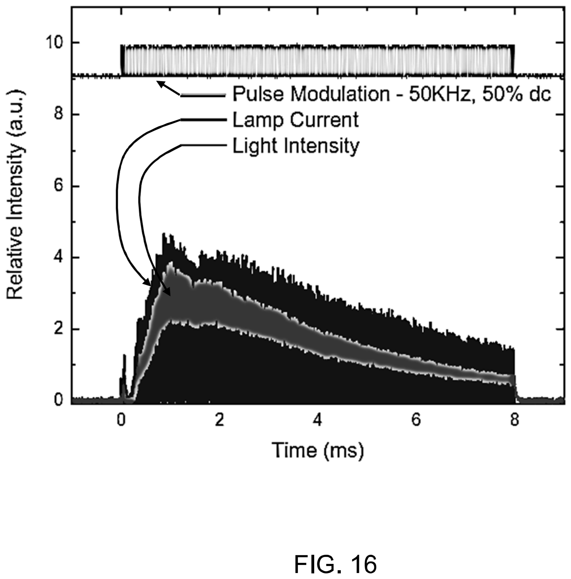

Fig. 16 shows the current and light output during a 50 kHz, 50% duty cycle over a long time scale;

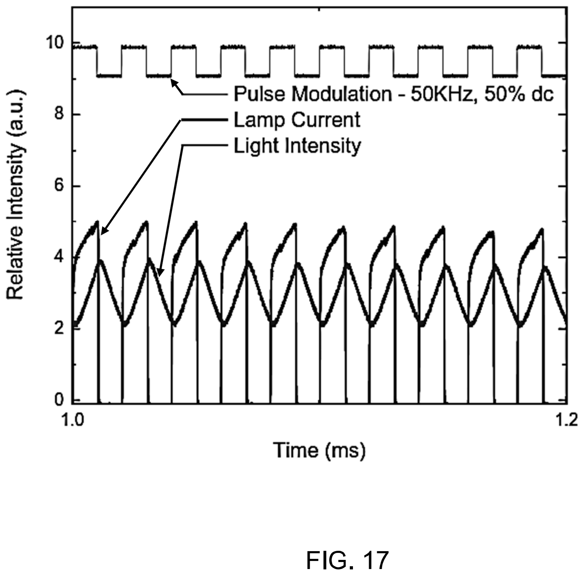

Fig. 17 shows the current and light output during a 50 kHz, 50% duty cycle over a short time scale;

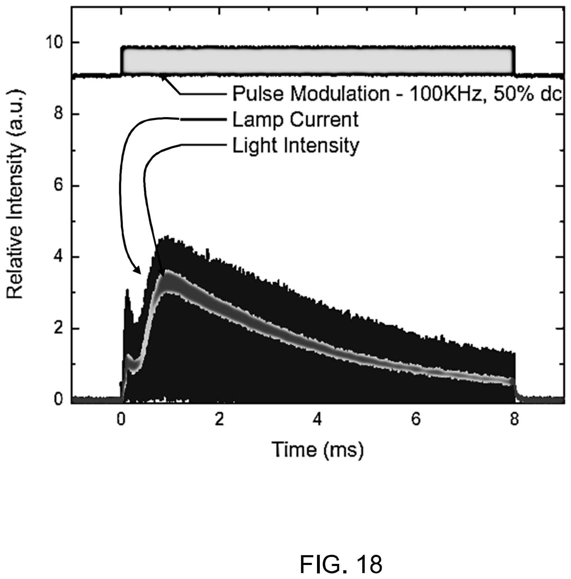

Fig. 18 shows the current and light output during a 100 kHz, 50% duty cycle over a long time scale;

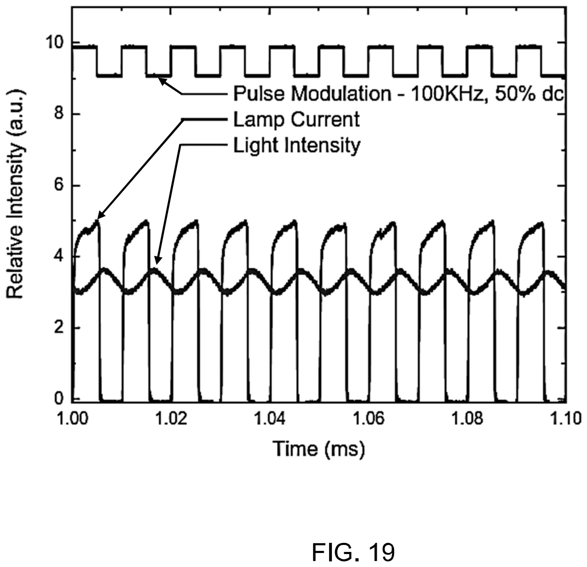

Fig. 19 shows the current and light output during a 100 kHz, 50% duty cycle over a short time scale;

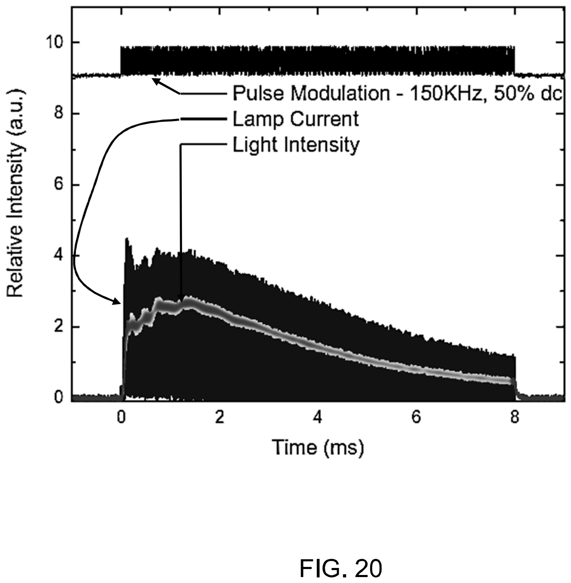

Fig. 20 shows the current and light output during a 150 kHz, 50% duty cycle over a long time scale;

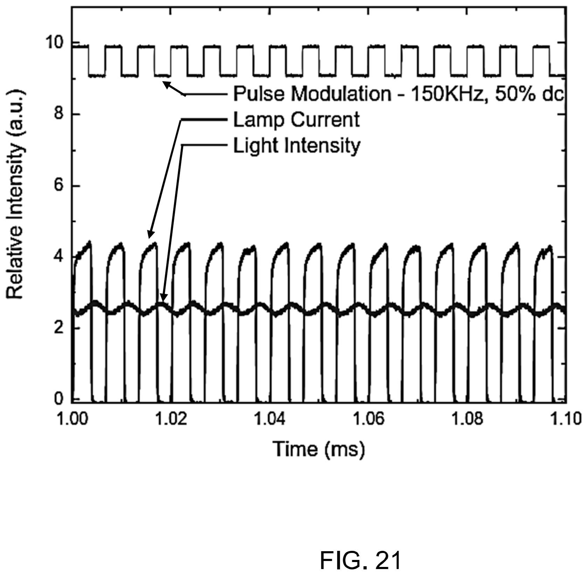

Fig. 21 shows the current and light output during a 150 kHz, 50% duty cycle over a short time scale;

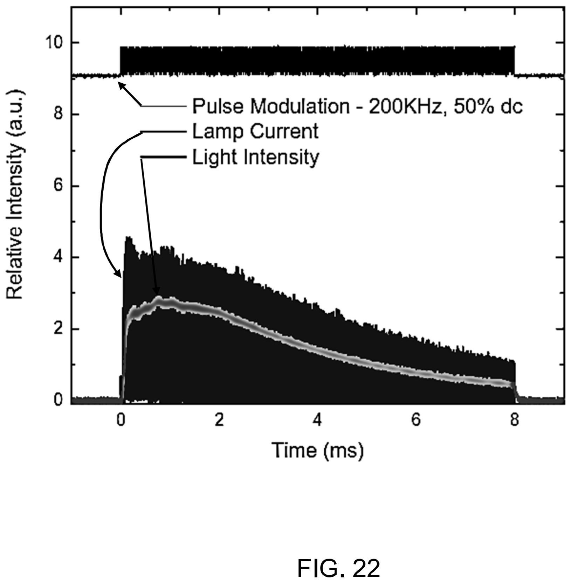

Fig. 22 shows the current and light output during a 200 kHz, 50% duty cycle over a long time scale;

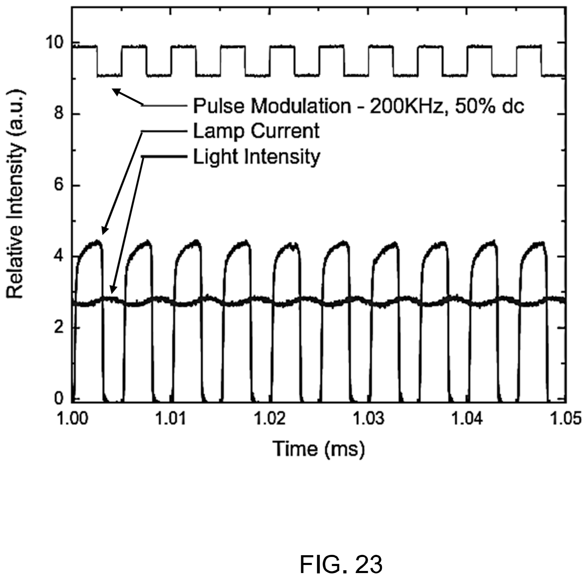

Fig. 23 shows the current and light output during a 200 kHz, 50% duty cycle over a short time scale;

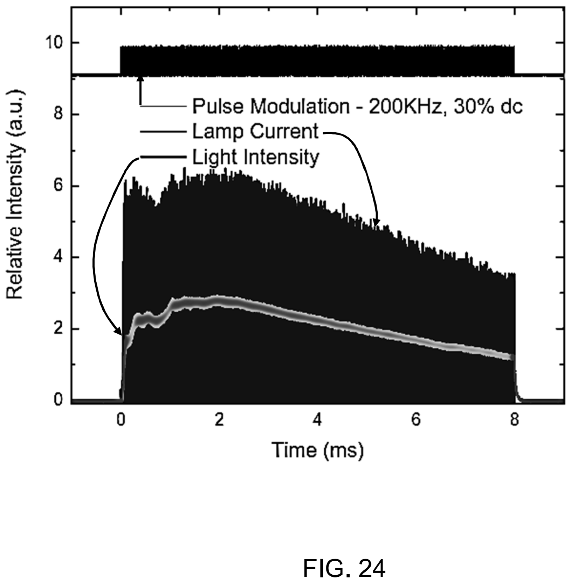

Fig. 24 shows the current and light output during a 200 kHz, 30% duty cycle over a long time scale;

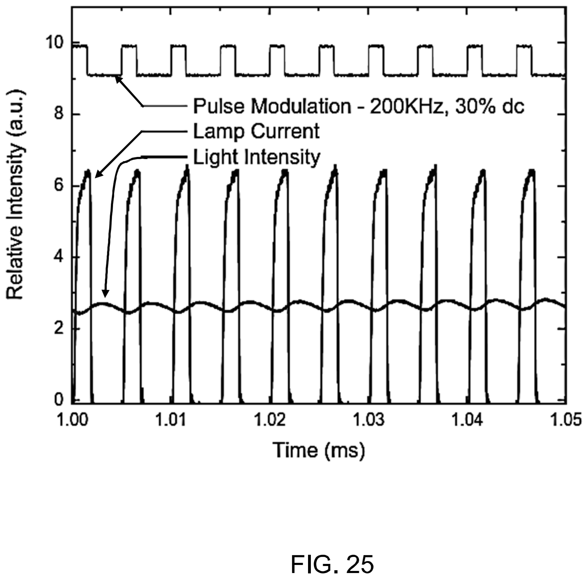

Fig. 25 shows the current and light output during a 200 kHz, 30% duty cycle modulated free decay discharge of an IPL device over a short time scale;

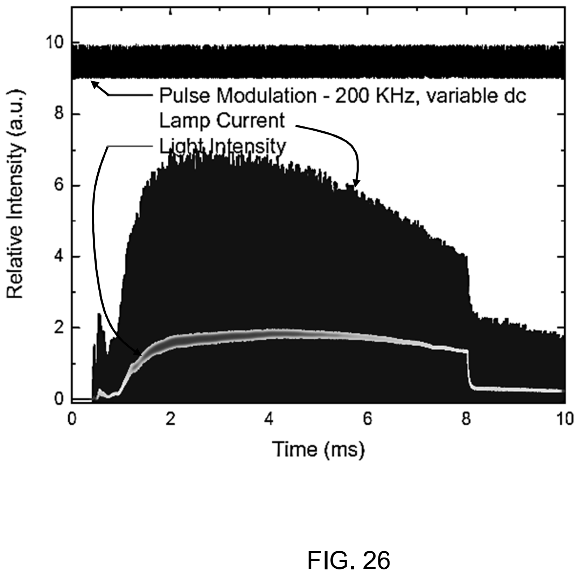

Fig. 26 shows the current and light output during a 200 kHz modulation, with variable duty cycle, over a long time scale;

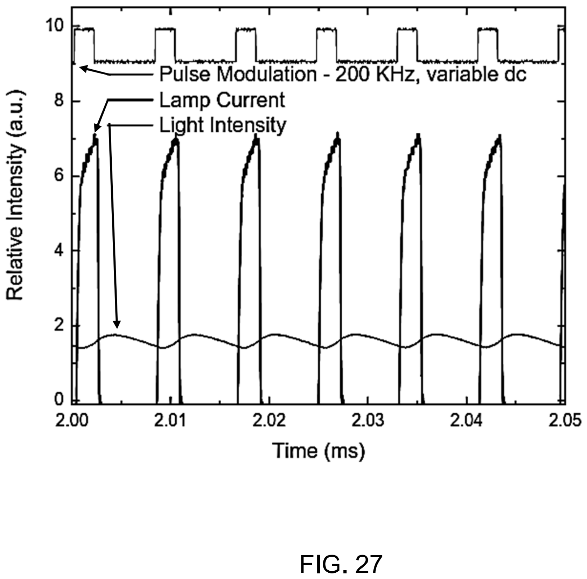

Fig. 27 shows the current and light output during the 200 kHz, modulation with variable duty cycle showing the beginning of an 8 ms pulse with a low duty cycle (30%);

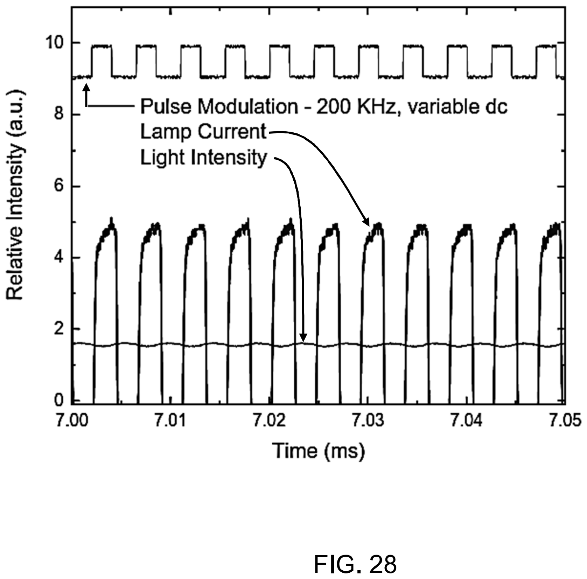

Fig. 28 shows the current and light output during the 200 kHz, modulation with variable duty cycle showing the end of the 8 ms pulse with a large duty cycle (50%);

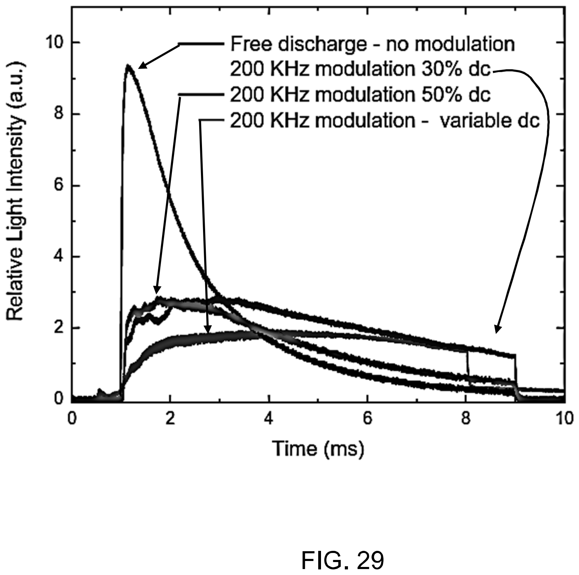

Fig. 29 shows the light intensity output with no modulation and with 200 kHz modulation with various duty cycles;

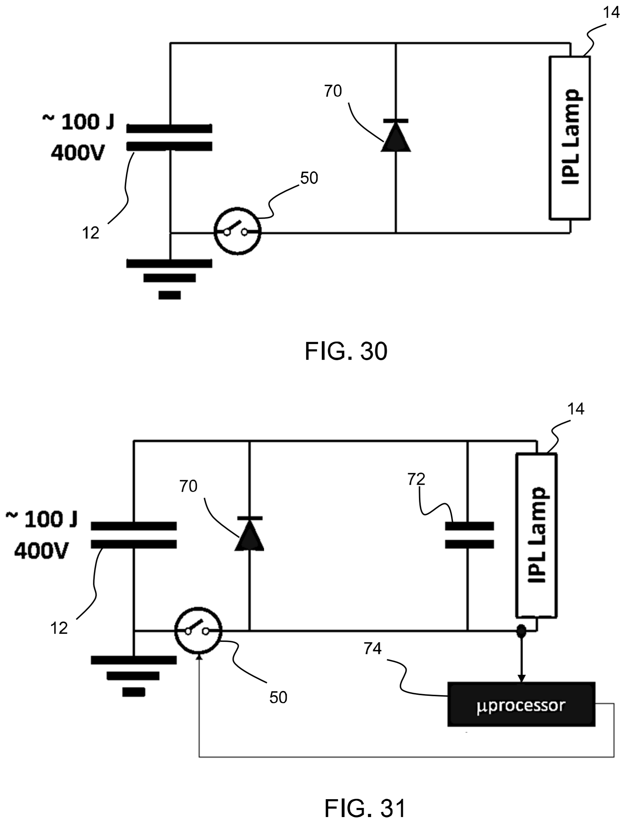

Fig. 30 shows a modification to the basic arrangement of Fig. 7 with an additional diode in parallel with the lamp;
Fig. 31 shows a modification in which a secondary capacitor is added in parallel with the lamp;

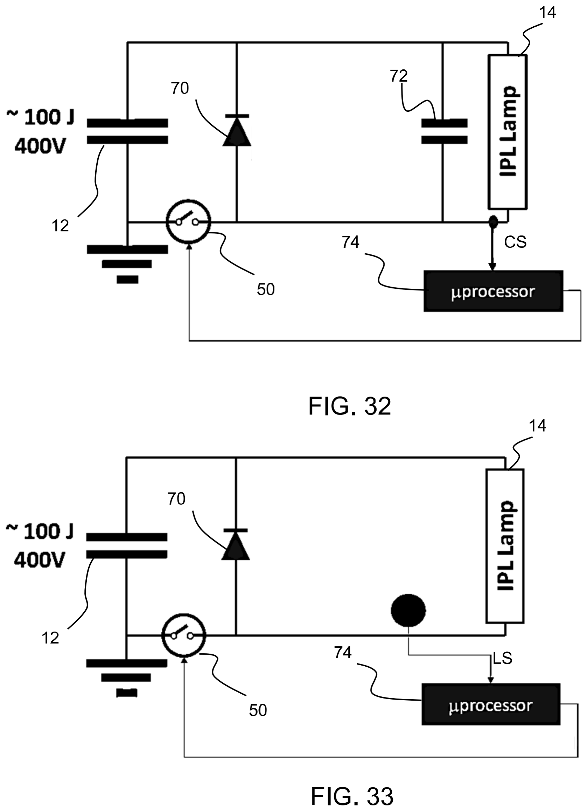

Fig. 32 shows the controller of Fig. 31 regulating the modulation based on a current sensor input;
Fig. 33 shows the controller of Fig. 31 regulating the modulation based on a light intensity sensor input;

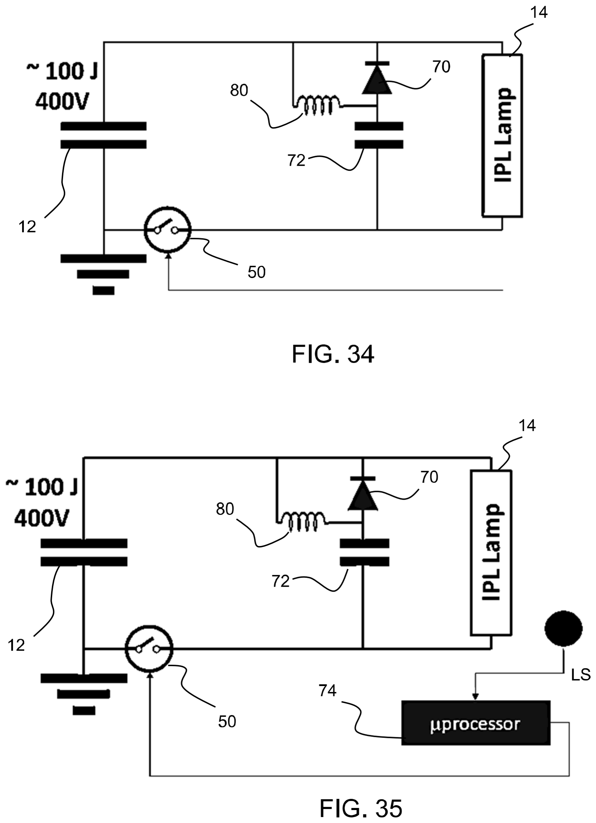

Fig. 34 shows a modification which includes a secondary capacitor, power diode and an inductor
Fig. 35 shows the controller of Fig. 34 regulating the modulation based on a light intensity sensor input; and

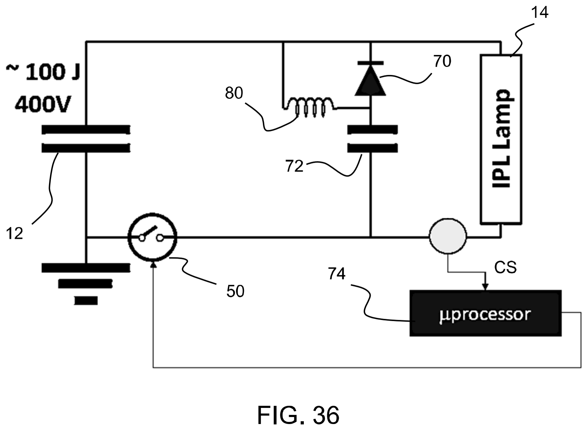

Fig. 36 shows the controller of Fig. 34 regulating the modulation based on a current sensor input.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a hair treatment device comprising an IPL lamp driven with pulses of energy. A switch is in series with the IPL lamp and is switched at a frequency in the range 50 kHz to 500 kHz to provide pulsed delivery of current to the IPL lamp with pulses shorter than the overall IPL pulse duration. The current modulation is used to eliminate the initial current peak and its associated peak in light output. The modulation can be used during the entire IPL pulse, or only during a fraction of it (in particular a first portion when the initial current peak is to be reduced), while the duty cycle or the frequency of the modulation can change during the IPL pulse in order to optimize the light output.

Fig. 5 shows a first example of the approach of the invention.

A switch 50 is provided in the circuit between the main capacitor 12 and the lamp 14. The switch is in series with the lamp and thereby allows or prevents current flowing to the lamp. The switch allows fast modulation of the IPL lamp current. The switch can for example comprise a power transistor able to modulate the large present current (which can exceed 100A) or any electrical or electronic device that allows the IPL lamp current to be modulated.

Fig. 6 shows one example where the switch is between the high voltage side of the capacitor and the lamp, and Fig. 7 shows the switch between the low voltage, ground, side of the capacitor and the lamp.

The drive circuitry, for example a plasma ignition unit and a booster capacitor used to start the discharge, are left out of the Figs. 5 to 7 for simplicity. However, these may be completely standard, for example as described above. No extra inductive (coil) or capacitive elements are used in these examples.

Fig. 8 shows the lamp current and light intensity (i.e. light output) during an unmodulated free decay discharge of an IPL device.

There is no pulse modulation, just a single square wave input to represent the time that the switch is on. The opening of the switch (e.g. IGBT transistor) marks the beginning of the discharge and the current peaks in a matter of microseconds (~45 microseconds) to its maximum value. The light output maximum is reached at about 130 microseconds after the opening of the IGBT transistor. The exact values when the current and the light output peak depends on the capacitor charge voltage, the transistor type and will show device to device deviations, but the important point is that the light intensity peak is always after the current peak in this type of free decay discharge IPL pulse.

After the initial current peak, the current value decays in an exponential manner, governed by the charge depletion stored in the capacitor. The light intensity follows the same trend, with a decaying intensity.

Fig. 9 shows the corresponding behavior of the device when a 1 kHz, 50% duty cycle modulation is introduced, using the configuration presented in Fig. 5. The IPL lamp current follows the modulation rather accurately due to a fast response time, while the light output does the same with some inertia. However, due to the 1 kHz modulation frequency, there is enough time for the current and light output to go to an almost zero value during the off part of the modulation. The overall envelope of the current and light intensity follows the form of a free decay that is interrupted and restarted.

As the frequency of modulation is increased, the IPL lamp current remains able to follow the modulation, while the light output is no longer able to follow the modulation.

Various modulation frequencies and some different duty cycles are illustrated in Figs. 10 to 25. The current modulation pulses are much shorter (with the second durations, e.g. of the order of µs) than the duration of the pulses of energy delivered by the IPL lamp by the drive circuit (the first duration, e.g. 8ms).

Fig. 10 shows the current and light output during a 5 kHz, 50% duty cycle modulated free decay discharge of an IPL device over a long time scale to show the overall performance. This corresponds to current pulse durations (the second durations) of 100µs.

Fig. 11 shows the current and light output during a 5 kHz, 50% duty cycle modulated free decay discharge of an IPL device, over a short time scale to show the performance at the level of the individual pulses.

Fig. 12 shows the current and light output during a 10 kHz, 50% duty cycle modulated free decay discharge of an IPL device over a long time scale. This corresponds to current pulse durations (the second durations) of 50µs.

Fig. 13 the current and light output during a 10 kHz, 50% duty cycle modulated free decay discharge of an IPL device over a short time scale.

Fig. 14 shows the current and light output during a 20 kHz, 50% duty cycle modulated free decay discharge of an IPL device over a long time scale. This corresponds to current pulse durations (the second durations) of 25µs.

Fig. 15 shows the current and light output during a 20 kHz, 50% duty cycle modulated free decay discharge of an IPL device over a short time scale.

Fig. 16 shows the current and light output during a 50 kHz, 50% duty cycle modulated free decay discharge of an IPL device over a long time scale. This corresponds to current pulse durations (the second durations) of 10µs.

Fig. 17 shows the current and light output during a 50 kHz, 50% duty cycle modulated free decay discharge of an IPL device over a short time scale.

Fig. 18 shows the current and light output during a 100 kHz, 50% duty cycle modulated free decay discharge of an IPL device over a long time scale. This corresponds to current pulse durations (the second durations) of 5µs.

Fig. 19 shows the current and light output during a 100 kHz, 50% duty cycle modulated free decay discharge of an IPL device over a short time scale.

Fig. 20 shows the current and light output during a 150 kHz, 50% duty cycle modulated free decay discharge of an IPL device over a long time scale. This corresponds to current pulse durations (the second durations) of 3.3µs.

Fig. 21 shows the current and light output during a 150 kHz, 50% duty cycle modulated free decay discharge of an IPL device over a short time scale.

Fig. 22 shows the current and light output during a 200 kHz, 50% duty cycle modulated free decay discharge of an IPL device over a long time scale. This corresponds to current pulse durations (the second durations) of 2.5µs.

Fig. 23 shows the current and light output during a 200 kHz, 50% duty cycle modulated free decay discharge of an IPL device over a short time scale.

Fig. 24 shows the current and light output during a 200 kHz, 30% duty cycle modulated free decay discharge of an IPL device over a long time scale. This corresponds to current pulse durations (the second durations) of 1.5µs.

Fig. 25 shows the current and light output during a 200 kHz, 30% duty cycle modulated free decay discharge of an IPL device over a short time scale.

The figures show that in the range 1 to 30 kHz, the light output shows a good modulation with low and high values, corresponding to the on and off states of the modulation. However, above 50 kHz, the light output modulation is minimal as the light no longer has time to follow the modulation of the current. The overall envelope of the light output has very little modulation, and importantly it no longer features the large initial peak, responsible for the user discomfort. The light intensity slowly increases towards its maximum and once that is reached it begins to slowly decay.

Fig. 26 shows the current and light output during a 200 kHz modulation, with variable duty cycle modulated free decay discharge of an IPL device, over a long time scale.

Fig. 27 shows the current and light output during the 200 kHz, modulation with variable duty cycle showing the beginning of the 8 ms pulse with a low duty cycle (30%).

Fig. 28 shows the current and light output during the 200 kHz, modulation with variable duty cycle showing the end of the 8 ms pulse with a large duty cycle (50%). Thus, in the examples of Figs. 27 and 28, the duty cycle is ramped over time from 30% to 50%. More generally, the duty cycle is ramped or stepped up from a lower value (at which the elimination of the current peak is most effective) to a higher value. The higher value may be 100% so that there is a pulsed delivery of current to the IPL lamp during a first portion of the pulses of energy and a continuous delivery of current during a last portion of the pulses of energy. In such a case, the modulation can be used not only for the full duration of the pulse, but it can also be used for a fraction of the pulse.

Fig. 29 shows examples of the light intensity output of the free discharge with no modulation and also the free discharge with 200 kHz modulation and various duty cycles, namely 30%, 50% and a ramp from 30% to 50%. The sharp initial peak in the light output of the free decay is not present in the modulated curves, indicating that a fast modulation can mitigate the discomfort associated with the initial peak of the free decay pulse.

Fig. 30 shows an additional example as a modification to the basic arrangement of Fig. 7. An additional power diode 70 is provided in parallel with the lamp, to make use of any residual inductance that the lamp might feature during the modulation.

Fig. 31 shows a further modification in which a secondary capacitor 72 is added in parallel with the lamp. The secondary capacitor can charge while the main capacitor output is on, while discharging over the lamp when the main capacitor is off.

A controller 74 (microprocessor) is also shown for regulating the modulation frequency and/or duty cycle, based on input from a current sensor or a light intensity sensor.

Fig. 32 shows the controller regulating the modulation based on a current sensor input CS and Fig. 33 shows the controller regulating the modulation based on a light intensity sensor input LS.

The examples above avoid the need for inductive components to perform the primary current smoothing function. However, circuit optimization may result in the use of small inductors. Figs. 34 to 36 show circuit examples which include the additional use of an inductor.

Fig. 34 shows a secondary capacitor 72 and power diode 70 in series and together in parallel with the lamp. An inductor 80 is connected between the high voltage terminal of the lamp and the node between the secondary capacitor and the power diode. The secondary capacitor can again charge while the main capacitor output is on, while discharging over the lamp when the main capacitor is off.

As explained above, the main capacitor output is modulated in the 50 kHz to 300 kHz regime, to eliminate the initial current peak and its associated peak in light output. The modulation can be used during the entire IPL pulse, or only during a faction of it, while the duty cycle or the frequency of the modulation can change during the IPL pulse in order to optimize the light output.

The secondary capacitor is smaller than the main capacitor, for example with a capacitance below 1% of the capacitance of the main capacitor. If the secondary capacitor is too large, the reduction in the initial current peak is suboptimal. The inductor has a small inductance (of the order of mH).

Fig. 35 shows the addition of a controller and light intensity sensor (in the same way as Fig. 33) and Fig. 36 shows the addition of a controller and current sensor (in the same way as Fig. 32).

By way of example, the main capacitor has a capacitance of the order of mF, such as 0.5 mF to 10 mF, for example 1.4 mF. The secondary capacitor for example has a capacitance of the order of µF, such as 10 µF to 1 mF, such as 22 µF.

The added inductor for example has an inductance of the order of mH, such as 0.5 mH to 10 mH, for example 5 mH.

The duty cycle and the frequency for the pulsed delivery of current will be selected based on the lamp details and circuit components (such as the main capacitor). Device to device differences are small such that a different devices will be tuned to the same parameters. During the life time of a device, parameter changes are typically larger than from device to device, as the optical output of the lamp decreases with time. These age-related changes can optionally be at least partially compensated by having controllable duty cycle and/or frequency, controlled using a feedback system which monitors the lamp performance over time.

The examples above make use of a current modulation to eliminate the initial current peak and its associated peak in light output, and thereby reduce user discomfort. This may be needed only for sensitive skin areas (e.g. the armpits), whereas less sensitive areas (e.g. the legs) may not need the current modulation and instead allow for more rapid delivery of energy. The device may therefore have different modes of operation; a (first) modulated current mode and a (second) normal mode without current modulation. This second mode then corresponds to a conventional mode of operation.

The device may therefore be operable in these two different modes, and allow the user to switch between the modes. The device may even switch between the modes automatically based on detection of the region of the body to which the device is being applied. This may be detected using motion/orientation sensing and/or camera image analysis.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A hair treatment device comprising:
an intense pulsed light, IPL, lamp (14);
a drive circuit (10, 12) for driving the IPL lamp with pulses of energy with first duration, wherein the drive circuit comprises an ignition unit (12) and a main storage capacitor;
a switch (50) in series with the IPL lamp; and
a controller (74) for controlling the switch at a frequency in the range 50 kHz to 500 kHz thereby to provide pulsed delivery of current to the IPL lamp, during at least a portion of the pulses of energy, with current delivery pulses of a second duration shorter than the first duration,
wherein the controller (74) is configured to increase the duty cycle from a first value in the range 20% to 40% to a second value in the range 40% to 70% over time during the pulses of energy.

2. The hair treatment device of claim 1, wherein the controller (74) is for controlling the switch at a frequency in the range 100 kHz to 300 kHz, for example 150kHz to 300kHz, for example 150kHz to 250kHz.

3. The hair treatment device of claim 1 or 2, wherein the first duration is in the range 5 ms to 10 ms.

4. The hair treatment device of any one of claims 1 to 3, wherein the controller (74) is configured to adjust the frequency of the control of the switch (50) over time during the pulses of energy.

5. The hair treatment device of any one of claims 1 to 4, wherein the controller (74) is configured to adjust a duty cycle of the control of the switch (50) over time during the pulses of energy.

6. The hair treatment device of any one of claims 1 to 5, further comprising a diode (70) in parallel with the IPL lamp with the anode of the diode connected to a low voltage terminal of the IPL lamp and the cathode of the diode connected to a high voltage terminal of the IPL lamp.

7. The hair treatment device of any one of the preceding claims, further comprising a secondary capacitor (72) in parallel with the IPL lamp.

8. The hair treatment device of claim 7, wherein the secondary capacitor (72) has a capacitance below 1% of the capacitance of the main capacitor.

9. The hair treatment device of any one of claims 1 to 5, further comprising a diode (70) and a secondary capacitor (72) in series with each other and together in parallel with the IPL lamp, and an inductor (80) between a high voltage terminal of the IPL lamp and a junction between the diode (70) and the secondary capacitor (72).

10. The hair treatment device of claim 9, wherein the secondary capacitor (72) has a capacitance below 1% of the capacitance of the main capacitor and the inductor has an inductance in the range 1 mH to 100 mH.

11. The hair treatment device of any one of claims 1 to 10, wherein the IPL lamp comprises a flash lamp with a fluence below 6.5 J/cm².

12. The hair treatment device of any one of claims 1 to 11 further comprising a current sensor and/or a light intensity sensor for providing a feedback signal to the controller.

13. The hair treatment device of any one of claims 1 to 12 wherein the controller is configurable to operate the device in first and second modes, a first mode with said current delivery pulses during said pulses of energy, and a second mode with delivery of continuous current during said pulses of energy.
